# EUROPEAN PATENT APPLICATION

(11) **EP 4 458 133 A1**
(43) Date of publication of application: **06.11.2024**
(21) Application number: 22913039.8
(22) Date of filing: 14.04.2022
(51) Int. Cl.: A01G 18/20, A01G 18/40, B65D 65/38, C12N 1/14

(54) **MYCELIUM PACKAGING MATERIAL HAVING ANTI-COUNTERFEITING FUNCTION AND METHOD FOR USING SAME**

(30) Priority: 31.12.2021 CN 202111671328
(71) Applicant: Shenzhen Zeqingyuan Technology Development Service Co., Ltd, Shenzhen, Guangdong 518000 (CN)
(72) Inventor: PENG, Zhili, Hong Kong Cooperation Zone, (Anchor At Shenzhen Qianhai Business Secretary Co., Ltd.) Shenzhen, Guangdong 518000 (CN); HUANG, Mengting, Hong Kong Cooperation Zone, (Anchor At Shenzhen Qianhai Business Secretary Co., Ltd.) Shenzhen, Guangdong 518000 (CN); CHEN, Jinfeng, Hong Kong Cooperation Zone, (Anchor At Shenzhen Qianhai Business Secretary Co., Ltd.) Shenzhen, Guangdong 518000 (CN)
(74) Representative: Petraz, Gilberto Luigi
(86) International application number: PCT/CN2022/086744
(87) International publication number: WO 2023/123737

(57) **Abstract**

A mycelium packaging material having an anti-counterfeiting function, which is formed by growing and twisting a culture material. The culture material is formed by mixing a password component, a basidiomycete strain and a biological raw material. The biological raw material comprises: 25-80 parts by weight of a wood fiber, 1-10 parts by weight of an auxiliary component, 5-35 parts by weight of a nutritional component and water. The auxiliary component is composed of 1-5 parts by weight of quicklime and 1-5 parts by weight of gypsum. The nutritional component is composed of 5-25 parts by weight of wheat bran and 0-15 parts by weight of corn starch. The addition amount of water is 1.2-1.4 times the total weight of the wood fiber, the auxiliary component and the nutritional component. The password component can be detected in the mycelium packaging material having an anti-counterfeiting function, and the addition amount of the password component is 0.1-10% of the total weight of the biological raw material. The mycelium packaging material has good compression performance, cushion performance and anti-counterfeiting function, and whether the subject matter is a certified product can be determined according to the appearance and the physical performance and by detecting whether a password component is comprised. The present application further relates to a method for using the mycelium packaging material having an anti-counterfeiting function.

## Description

The present application claims the benefit of Chinese Patent Application No. 202111671328.2 filed on December 31, 2021, the contents of which are incorporated herein by reference in their entirety.

### Technical Field

The present application relates to the technical field of anti-counterfeiting packaging, in particular to a mycelium packaging material having an anti-counterfeiting function and a method for using the same.

### Background Art

An anti-counterfeiting technology refers to measures taken to achieve the purpose of anti-counterfeiting, which can accurately identify the authenticity within a certain range and is not easy to be imitated and copied. Anti-counterfeiting is a preventive measure taken for imitation or copying activities that are based on deception and cannot be permitted by owners, and also a preventive technical measure taken to protect the corporate brand, protect the market and protect the legitimate rights and interests of consumers.

There are many types of anti-counterfeiting technologies, mainly including printing anti-counterfeiting technologies, chemical material anti-counterfeiting technologies, physical anti-counterfeiting technologies, digital information anti-counterfeiting technologies, structure and packaging anti-counterfeiting technologies, human body and biological characteristic anti-counterfeiting technologies, etc. The chemical material anti-counterfeiting technologies are easy for consumers to identify, but also easy to be imitated in batches. The printing anti-counterfeiting and physical laser anti-counterfeiting technologies are of common anti-counterfeiting, which are low in cost but easy to be imitated in appearance in batches, and also relatively difficult for consumers to identify. The digital information anti-counterfeiting technologies are of personalized anti-counterfeiting, which are relatively simple for consumers to identify, but easy to be imitated in appearance, and have hidden dangers and defects of code theft, code copying and batch copying, and also low query rate. The structure and packaging anti-counterfeiting technologies are based on inherent stripe marks of a packaging material itself as anti-counterfeiting identification marks, and also of personalized anti-counterfeiting technologies, which are extremely difficult to imitate, but the form of expression thereof is limited to labels. The human body and biological characteristic anti-counterfeiting technologies are of a novel anti-counterfeiting technology, which is developed by the use of human or other biological characteristics, immunity, DNA detection and other technical means, and has efficient and high-quality anti-counterfeiting ability, but with the help of instruments for authenticity identification, is not suitable for the field of commodity anti-counterfeiting.

### Summary of the Invention

The technical problem to be solved by the present application is to design a new low-cost anti-counterfeiting method that is easy to be identified by consumers and is difficult to be imitated in batches.

In order to solve the above technical problems, the present application adopts the following technical solutions.

A mycelium packaging material having an anti-counterfeiting function is formed by growing and twisting a culture material, wherein the culture material is formed by mixing a password component, a basidiomycete strain and a biological raw material; the biological raw material includes: 25-80 parts by weight of a wood fiber, 1-10 parts by weight of an auxiliary component, 5-35 parts by weight of a nutritional component and water; the auxiliary component consists of 1-5 parts by weight of quicklime and 1-5 parts by weight of gypsum; the nutritional component consists of 5-25 parts by weight of wheat bran and 0-15 parts by weight of com flour; the addition amount of water is 1.2-1.4 times the total weight of the wood fiber, the auxiliary component and the nutritional component; and the password component can be detected in the mycelium packaging material having the anti-counterfeiting function, and the addition amount of the password component is 0.1-10% of the total weight of the biological raw material.

Further, the password component is one or more of a plant or a plant extract; the plant is a food-induced and/or drug-induced plant, and has a particle size of 0-1 cm; the plant extract is a food-induced and/or drug-induced plant extract; and the addition amount of the password component is 0.5-5% of the total weight of the biological raw material.

Further, the plant is one or more of green tea, black tea, yellow tea, white tea, dark green tea, black tea, Fagopyrum tataricum, Folium Nelumbinis, Phyllostachys nigra, Taxus chinensis, Armoracia rusticana, Camptotheca acuminata, Catharanthus roseus, Rauvolfia verticillata, Styrax benzoin, Fructus Anisi Stellatim, Rhizoma Cynanchi Stauntonii, Radix Paeoniae Alba, Atractylodes macrocephala, Pulsatilla chinensis, Cortex Dictamni, Radix Angelicae Dahuricae, Radix Isatidis, Fructus Piperis Longi, Herba Polygoni Avicularis, Areca catechu, Herba Menthae, Psoraleae Fructus, Atractylodes Lancea, Alpinia katsumadai Hayata, Folium Aconiti Kusnezoffii, Radix Bupleuri, Semen Plantaginis, Pericarpium Citri Reticulatae, Radix Paeoniae Rubra, Caulis Clematidis Armandii, Radix Vladimiriae, Herba Andrographitis, Ailanthi Cortex, Acanthopanax senticosus, Paris polyphylla, Radix et Rhizoma Rhei, Radix Cirsii Japonici, Folium Isatidis, Caulis Sargentodoxae, Cortex Illicii, Fructus Kochiae Scopariae, Radix Rehmanniae, Radix Sanguisorbae, Herba Belladonnae, Caulis Erycibes, Syringa vulgaris, Malvae Fructus, Alpinia katsumadai Hayata, Angelicae Pubescentis Radix, Cortex Eucommiae, Clinopodii Herba, Radix Changii, Folium Sennae, Radix Saposhnikoviae, Radix Stephaniae Tetrandrae, Rhizoma Dioscoreae Septemlobae, Fructus Citri Sarcodactylis, Poria cocos, Rhizoma Zingiberis, Radix Glycyrrhizae, Rhizoma Ligustici, Radix Puerariae Lobatae, Ramulus Uncariae Cum Uncis, Rhizoma Drynariae, Pericarpium Trichosanthis, Desmodium styracifolium, Cinnamomi Ramulus, seaweed, Fructus Chebulae, Albizia julibrissin, Fallopia multiflora, Ginseng Radix Rubra, Rhodiola rosea, Radix knoxiae, Fructus Galangae, Flos Carthami, Hedysari Radix, Cortex Magnoliae Officinalis, Rhizoma Picrorhizae, Pipemigrum, Bulbus Fritillariae Hupehensis, Semen Trigonellae, Viscum coloratum, Rhizoma Polygoni Cuspidati, Zanthoxylum bungeanum Maxim, Citri Grandis Exocarpium, Flos Sophorae, Phellodendri Chinensis Cortex, Rhizoma Coptidis, Radix Astragali, Radix Scutellariae, Fructus Cannabis, Celosia cristata, Herba Abri, Rhizoma Curcumae Longae, Lignum Dalbergiae Odoriferae, Gardeniae Fructus Praeparatus, Sinapis Semen, Radix Tinosporae, Fagopyri Dibotryis Rhizoma, Lonicera Japanica Thunb, Rosae Laevigatae Fructus, Calyx seu Fructus Physalis, Schizonepetae Herba, Murraya exotica, Platycodonis Radix, Flos Chrysanthemi, Exocarpium Citri Rubrum, Herba Selaginellae, Semen Cassiae, Radix Sophorae Fiavescentis, Thallus Laminariae, Semen Raphani, Glechoma longituba, Forsythia suspensa, Receptaculum Nelumbinis, Semen Nelumbinis, Zanthoxylum nitidum, Folium polygoni Tinctorll, Campsis grandiflora, Rhapontici Radix, Rhizoma Phragmitis, Fructus Momordicae, Herba Ephedrae, Verbena officinali, Portulacae Herba, Aristolochia debilis, Rhododendri Daurici Folium, Fructus Viticis, Terminalia chebula, Cortex Moutan, Herba Echiptae, Chaenomelis Fructus, Semen Oroxyli, Radix Aucklandiae, Radix et Rhizoma Baphicacanthi, Radix Adenophorae, Fructus Schisandrae Sphenantherae, Fructus Arctii, Achyranthes bidentata Blume, Semen Sterculiae Lychnophorae, Eupatorii Herba, Taraxacum mongolicum, Pollen Typhae Angustifoliae, Herba Dianthi, Homalomena occulta, Radix Peucedani, Radix Rubiae, Radix Gentianae Macrophyllae, Cortex Fraxini, Indigo Naturalis, Caulis Sinomenii, Artemisia carvifolia, Radix Aucklandiae, Pericarpium Citri Reticulatae Viride, Herba Swertiae Mileensis, Rhizoma Bistortae, Ginseng Radix et Rhizoma, Folium Ginseng, Herba cistanches, Cinnamomum cassia Presl, Saururus chinensis, Notoginseng Radix Et Rhizoma, cortex mori, Herba Taxilli, Mori Folium, Hippophae, Semen Astragali Complanati, Rhizoma Kaempferiae, Rhizoma Belamcandae, Herba Lycopodii, Cimicifugae Rhizoma, Rhizoma Acori Tatarinowii, Pericarpium Granati, Calyx Kaki, Polygoni Multiflori Caulis, Lignum Sappan, Semen Ziziphi spinosae, Herba Cynomorii, Radix Pseudstellariae, Trichosanthis Radix, Radix Semiaquilegiae, Bulbus Fritillariae Thunbergii, Radix Inulae Helenii, Santali Albi Lignum, Potentillae Chinensis Herba, Radix Linderae, Croci Stigma, Radix Panacis Quinquefolii, Spica Prunellae, Corydafis Decumbentis Rhizoma, Cyperi Rhizoma, Foeniculi Fructus, Herba Cirsii Setosi, Bulbus Allii Macrostemonis, Flos Magnoliae, Radix Cynanchi Paniculati, Flos Inulae, Herba Cissampelotis, Linum usitatissimum, Rhizoma Corydalis, Flos Chrysanthemi Indici, Fritillariae Pallidiflorae Bulbus, Leonuri Herba, Herba Epimedii, Radix Stellariae, Folium Ginkgo, Houttuynia cordata Thunb, Pruni Semen, Polygalae Radix, Rhizoma Acori Calami, Bulbus Fritillariae Thunbergii, Rhizoma Anemarrhenae, Gardeniae Fructus, Aurantii Fructus, Fructus Aurantii Immaturus, Sarcandrae Herba, Rhizoma panacis majoris, Rhizoma Panacis Japonici, Radix Amebiae, Herba Violae, Folium Perillae and Trachycarpus fortunei; and the plant extract is one or more of Armoracia rusticana powder, locust bean gum, guar gum, a green tea extract, a black tea extract, a yellow tea extract, a white tea extract, a dark green tea extract, a black tea extract, a Fagopyrum tataricum extract, a Folium Nelumbinis extract, a Phyllostachys nigra extract, a Taxus chinensis extract, a Camptotheca acuminata extract, a Catharanthus roseus extract, a Rauvolfia verticillate extract, a Styrax benzoin extract, a Fructus Anisi Stellatim extract, a Rhizoma Cynanchi Stauntonii extract, a Radix Paeoniae Alba extract, an Atractylodes macrocephala extract, a Pulsatilla chinensis extract, a Cortex Dictamni extract, a Radix Angelicae Dahuricae extract, a Radix Isatidis extract, a Fructus Piperis Longi extract, a Herba Polygoni Avicularis extract, an Areca catechu extract, a Herba Menthaeextract, a Psoraleae Fructus extract, an Atractylodes Lancea extract, a Alpinia katsumadai Hayata extract, a Folium Aconiti Kusnezoffii extract, a Radix Bupleuri extract, a Semen Plantaginis extract, a Pericarpium Citri Reticulatae extract, a Radix Paeoniae Rubra extract, a Caulis Clematidis Armandii extract, a Radix Vladimiriae extract, a Herba Andrographitis extract, an Ailanthi Cortex extract, an Acanthopanax senticosus extract, a Paris polyphylla extract, a Radix et Rhizoma Rhei extract, a Herba Cirsii Japonici Radix Cirsii Japonici extract, a Folium Isatidis extract, a Caulis Sargentodoxae extract, a Cortex Illicii extract, a Fructus Kochiae Scopariae extract, a Radix Rehmanniae extract, a Radix Sanguisorbae extract, a Herba Belladonnae extract, a Caulis Erycibes extract, a Syringa vulgaris extract, a Malvae Fructus extract, an Alpinia katsumadai Hayata extract, an Angelicae Pubescentis Radix extract, a Cortex Eucommiae extract, a Clinopodii Herba extract, a Radix Changii extract, a Folium Sennae extract, a Radix Saposhnikoviae extract, a Radix Stephaniae Tetrandrae extract, a Rhizoma Dioscoreae Septemlobae extract, a Fructus Citri Sarcodactylis extract, a Poria cocos extract, a Rhizoma Zingiberis extract, a Radix Glycyrrhizae extract, a Rhizoma Ligustici extract, a Radix Puerariae Lobatae extract, a Ramulus Uncariae Cum Uncis extract, a Rhizoma Drynariae extract, a Pericarpium Trichosanthis extract, a Desmodium styracifolium extract, a Cinnamomi Ramulus extract, a seaweed extract, a Fructus Chebulae extract, an Albizia julibrissin extract, a Fallopia multiflora extract, a Ginseng Radix Rubra extract, a Rhodiola rosea extract, a Radix knoxiae extract, a Fructus Galangae extract, a Flos Carthami extract, a Hedysari Radix extract, a Cortex Magnoliae Officinalis extract, a Rhizoma Picrorhizae extract, a Pipemigrum extract, a Bulbus Fritillariae Hupehensis extract, a Semen Trigonellae extract, a Viscum coloratum extract, a Rhizoma Polygoni Cuspidati extract, a Zanthoxylum bungeanum Maxim extract, a Citri Grandis Exocarpium extract, a Flos Sophorae extract, a Phellodendri Chinensis Cortex extract, a Rhizoma Coptidis extract, a Radix Astragali extract, a Radix Scutellariae extract, a Fructus Cannabis extract, a Celosia cristata extract, a Herba Abri extract, a Rhizoma Curcumae Longae extract, a Lignum Dalbergiae Odoriferae extract, a Gardeniae Fructus Praeparatus extract, a Sinapis Semen extract, a Radix Tinosporae extract, a Fagopyri Dibotryis Rhizoma extract, a Lonicera JapanicaThunb extract, a Rosae Laevigatae Fructus extract, a Calyx seu Fructus Physalis extract, a Schizonepetae Herba extract, a Murraya exotica extract, a Platycodonis Radix extract, a Flos Chrysanthemi, Exocarpium Citri Rubrum extract, a Herba Selaginellae extract, a Semen Cassiae extract, a Radix Sophorae Fiavescentis extract, a Thallus Laminariae extract, a Semen Raphani extract, a Glechoma longituba extract, a Forsythia suspensa extract, a Receptaculum Nelumbinis extract, a Semen Nelumbinis extract, a Zanthoxylum nitidum extract, a Folium polygoni Tinctorll extract, a Campsis grandiflora extract, a Rhapontici Radix extract, a Rhizoma Phragmitis extract, a Fructus Momordicae extract, a Herba Ephedrae extract, a Verbena officinali extract, a Portulacae Herba extract, an Aristolochia debilis extract, a Rhododendri Daurici Folium extract, a Fructus Viticis extract, a Terminalia chebula extract, a Cortex Moutan extract, a Herba Echiptae extract, a Chaenomelis Fructus extract, a Semen Oroxyli extract, a Radix Aucklandiae extract, a Radix et Rhizoma Baphicacanthi extract, a Radix Adenophorae extract, a Fructus Schisandrae Sphenantherae extract, a Fructus Arctii extract, an Achyranthes bidentata Blume extract, a Semen Sterculiae Lychnophorae extract, a Eupatorii Herba extract, a Taraxacum mongolicum extract, a Pollen Typhae Angustifoliae extract, a Herba Dianthi extract, a Homalomena occulta extract, a Radix Peucedani extract, a Radix Rubiae extract, a Radix Gentianae Macrophyllae extract, a Cortex Fraxini extract, an Indigo Naturalis extract, a Caulis Sinomenii extract, an Artemisia carvifolia extract, a Radix Aucklandiae extract, a Pericarpium Citri Reticulatae Viride extract, a Herba Swertiae Mileensis extract, a Rhizoma Bistortae extract, a Ginseng Radix et Rhizoma extract, a Folium Ginseng extract, a Herba cistanches extract, a Cinnamomum cassia Presl extract, a Saururus chinensis extract, a Notoginseng Radix Et Rhizoma extract, a cortex mori extract, a Herba Taxilli extract, a Mori Folium extract, a Hippophae extract, a Semen Astragali Complanati extract, a Rhizoma Kaempferiae extract, a Rhizoma Belamcandae extract, a Herba Lycopodii extract, a Cimicifugae Rhizoma extract, a Rhizoma Acori Tatarinowii extract, a Pericarpium Granati extract, a Calyx Kaki extract, a Polygoni Multiflori Caulis extract, a Lignum Sappan extract, a Semen Ziziphi spinosae extract, a Herba Cynomorii extract, a Radix Pseudstellariae extract, a Trichosanthis Radix extract, a Radix Semiaquilegiae extract, a Bulbus Fritillariae Thunbergii extract, a Radix Inulae Helenii extract, a Santali Albi Lignum extract, a Potentillae Chinensis Herba extract, a Radix Linderae extract, a Croci Stigma extract, a Radix Panacis Quinquefolii extract, a Spica Prunellae extract, a Corydalis Decumbentis Rhizoma extract, a Cyperi Rhizoma extract, a Foeniculi Fructus extract, a Herba Cirsii Setosi extract, a Bulbus Allii Macrostemonis extract, a Flos Magnoliae extract, a Radix Cynanchi Paniculati extract, a Flos Inulae extract, a Herba Cissampelotis extract, a Linum usitatissimum, Rhizoma Corydalis extract, a Flos Chrysanthemi Indici extract, a Fritillariae Pallidiflorae Bulbus extract, a Leonuri Herba extract, a Herba Epimedii extract, a Radix Stellariae extract, a Folium Ginkgo extract, a Houttuynia cordata Thunb extract, a Pruni Semen extract, a Polygalae Radix extract, a Rhizoma Acori Calami extract, a Bulbus Fritillariae Thunbergii extract, a Rhizoma Anemarrhenae extract, a Gardeniae Fructus extract, an Aurantii Fructus extract, a Fructus Aurantii Immaturus extract, a Sarcandrae Herba extract, a Rhizoma panacis majoris extract, a Rhizoma Panacis Japonici extract, a Radix Amebiae extract, a Herba Violae extract, a Folium Perillae extract and a Trachycarpus fortune extract.

Further, the basidiomycete strain is one of oyster mushroom, pleurotus nebrodensis, Ganodenna lucidum, shiitake mushroom, straw mushroom, enoki mushroom and trametes gibbosa, and has an inoculation amount of 10-30% of the total weight of the biological raw material; the wood fiber is one or more of hemp stalks, wheat straws, corn straws, miscellaneous sawdust, and soybean straws; the wood fiber has a particle size of 0-1 cm (excluding an endpoint value 0); and the wood fiber consists of 15-50 parts by weight of hemp stalks, 0-15 parts by weight of wheat straws, 15-40 parts by weight of miscellaneous sawdust, 0-35 parts by weight of corn straws, and 0-20 parts by weight of soybean straws.

A method for using the mycelium packaging material having the anti-counterfeiting function includes the following steps which are implemented in sequence:

S1: preparation of culture material: first preparing a biological raw material according to a formula, mixing well and then sterilizing; then inoculating a basidiomycete strain; and finally mixing a password component into the inoculated biological raw material, and mixing well to obtain the culture material; and

S2: molding culture: putting the culture material and a subject matter together in a breathable and waterproof sterile mold such that the culture material evenly wraps an outer wall of the subject matter, and finally culturing in the dark for 5-8 days under the conditions that the temperature is 20-30°C and the humidity is 90-100% to obtain an anti-counterfeiting subject matter.

Further, the method further includes a step S3:
S3: anti-counterfeiting detection: first observing whether a material around a suspected subject matter is smooth and flat, whether the color is uniform, whether a surface mycelium is white and dense, and whether the structure is intact; then detecting physical properties of the material around the suspected subject matter; and finally taking the material around the suspected subject matter (an amount of the taken material is related to the addition amount of the password component and an amount required in its detection method), selecting a corresponding detection method according to the password component added in the corresponding batch, and detecting whether the material contains the added password component.

Further, before the culture material wraps the outer wall of the subject matter, a filler block is placed on the outer wall of the subject matter corresponding to a trademark position; and after the culture is completed, the filler block is taken away.

Further, after the culture is completed, the culture material is also air-dried in natural air until the water content of the mycelium packaging material having the anti-counterfeiting function is less than 15%.

Further, during the preparation of the biological raw material, wood fiber and an auxiliary component are first mixed with water and then stacked and fermented for 12-24 h, and then added with a nutritional component.

Further, the biological raw material is autoclaved for 1-2 h under the conditions that the temperature is 121-126°C and the pressure is 0.12-0.15 MPa; and the password component is also sterilized before addition.

The mycelium packaging material having the anti-counterfeiting function of the present application has the following beneficial effects: it has good compression and cushioning performances, and plays a role in shock absorption and cushioning for the packaged subject matter; after the subject matter is unpacked, the mycelium packaging material cannot be restored; ordinary consumers can judge whether the subject matter is certified by means of indexes, such as whether the appearance of the material is smooth and flat, whether the color is uniform, whether the surface mycelium is white and dense, whether the structure is intact without damage, and whether it is not easy to be broken; and a manufacturer of the subject matter can choose to add different password components according to different batches, so that the mycelium packaging material is difficult to be copied, and at the same time, whether the subject matter is certified is judged by detecting whether the mycelium packaging material contains the password component correspondingly added in this batch. The mycelium packaging material is eco-friendly, and free of any harmful substances to the human body and the environment, can be decomposed by microorganisms in the soil about 90 days after landfilling, and can increase the soil fertility after degradation.

### Brief Description of the Drawings

The specific structure of the present application is described below in conjunction with the accompanying drawings.

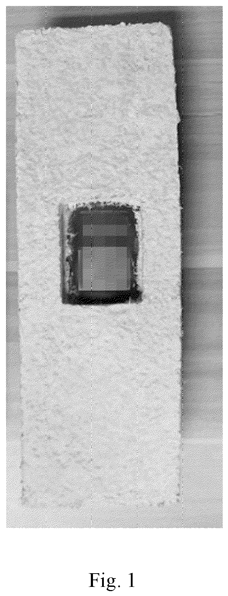

FIG. 1 is an effect diagram of an anti-counterfeiting subject matter which is obtained by packaging with a mycelium packaging material having an anti-counterfeiting function.

### Detailed Description of the Invention

In order to describe the technical content, structural characteristics and implementing objects and effects of the present application in detail, the following description is given with reference to the specific embodiments in combination with the accompanying drawings.

### Example 1

A mycelium packaging material having an anti-counterfeiting function is formed by growing and twisting a culture material, wherein the culture material is formed by mixing a password component, a basidiomycete strain and a biological raw material; the biological raw material includes: 25-80 parts by weight of a wood fiber, 1-10 parts by weight of an auxiliary component, 5-35 parts by weight of a nutritional component and water; the auxiliary component consists of 1-5 parts by weight of quicklime and 1-5 parts by weight of gypsum; the nutritional component consists of 5-25 parts by weight of wheat bran and 0-15 parts by weight of com flour; the addition amount of water is 1.2-1.4 times the total weight of the wood fiber, the auxiliary component and the nutritional component; the password component is one or more of a plant or a plant extract; the plant is a food-induced and/or drug-induced plant and has a particle size of 0-1 cm; and the plant extract is a food-induced and/or drug-induced plant extract, and the addition amount of the password component is 0.1-10% of the total weight of the biological raw material. The password component can be detected in the mycelium packaging material having the anti-counterfeiting function. The plant is one or more of black tea, yellow tea, white tea, dark green tea, black tea, Fagopyrum tataricum, Folium Nelumbinis, Phyllostachys nigra, Taxus chinensis, Armoracia rusticana, Camptotheca acuminata, Catharanthus roseus, Rauvolfia verticillata, Styrax benzoin, Fructus Anisi Stellatim, Rhizoma Cynanchi Stauntonii, Radix Paeoniae Alba, Atractylodes macrocephala, Pulsatilla chinensis, Cortex Dictamni, Radix Angelicae Dahuricae, Radix Isatidis, Fructus Piperis Longi, Herba Polygoni Avicularis, Areca catech, Herba Menthae, Psoraleae Fructus, Atractylodes Lancea, Alpinia katsumadai Hayata, Folium Aconiti Kusnezoffii, Radix Bupleuri, Semen Plantaginis, Pericarpium Citri Reticulatae, Radix Paeoniae Rubra, Caulis Clematidis Armandii, Radix Vladimiriae, Herba Andrographitis, Ailanthi Cortex, Acanthopanax senticosus, Paris polyphylla, Radix et Rhizoma Rhei, Herba Cirsii Japonici Radix Cirsii Japonici, Folium Isatidis, Caulis Sargentodoxae, Cortex Illicii, Fructus Kochiae Scopariae, Radix Rehmanniae, Radix Sanguisorbae, Herba Belladonnae, Caulis Erycibes, Syringa vulgaris, Malvae Fructus, Alpinia katsumadai Hayata, Angelicae Pubescentis Radix, Cortex Eucommiae, Clinopodii Herba, Radix Changii, Folium Sennae, Radix Saposhnikoviae, Radix Stephaniae Tetrandrae, Rhizoma Dioscoreae Septemlobae, Fructus Citri Sarcodactylis, Poria cocos, Rhizoma Zingiberis, Radix Glycyrrhizae, Rhizoma Ligustici, Radix Puerariae Lobatae, Ramulus Uncariae Cum Uncis, Rhizoma Drynariae, Pericarpium Trichosanthis, Desmodium styracifolium, Cinnamomi Ramulus, seaweed, Fructus Chebulae, Albizia julibrissin, Fallopia multiflora, Ginseng Radix Rubra, Rhodiola rosea, Radix knoxiae, Fructus Galangae, Flos Carthami, Hedysari Radix, Cortex Magnoliae Officinalis, Rhizoma Picrorhizae, Pipemigrum, Bulbus Fritillariae Hupehensis, Semen Trigonellae, Viscum coloratum, Rhizoma Polygoni Cuspidati, Zanthoxylum bungeanum Maxim, Citri Grandis Exocarpium, Flos Sophorae, Phellodendri Chinensis Cortex, Rhizoma Coptidis, Radix Astragali, Radix Scutellariae, Fructus Cannabis, Celosia cristata, Herba Abri, Rhizoma Curcumae Longae, Lignum Dalbergiae Odoriferae, Gardeniae Fructus Praeparatus, Sinapis Semen, Radix Tinosporae, Fagopyri Dibotryis Rhizoma, Lonicera JapanicaThunb, Rosae Laevigatae Fructus, Calyx seu Fructus Physalis, Schizonepetae Herba, Murraya exotica, Platycodonis Radix, Flos Chrysanthemi, Exocarpium Citri Rubrum, Herba Selaginellae, Semen Cassiae, Radix Sophorae Fiavescentis, Thallus Laminariae, Semen Raphani, Glechoma longituba, Forsythia suspensa, Receptaculum Nelumbinis, Semen Nelumbinis, Zanthoxylum nitidum, Folium polygoni Tinctorll, Campsis grandiflora, Rhapontici Radix, Rhizoma Phragmitis, Fructus Momordicae, Herba Ephedrae, Verbena officinali, Portulacae Herba, Aristolochia debilis, Rhododendri Daurici Folium, Fructus Viticis, Terminalia chebula, Cortex Moutan, Herba Echiptae, Chaenomelis Fructus, Semen Oroxyli, Radix Aucklandiae, Radix et Rhizoma Baphicacanthi, Radix Adenophorae, Fructus Schisandrae Sphenantherae, Fructus Arctii, Achyranthes bidentata Blume, Semen Sterculiae Lychnophorae, Eupatorii Herba, Taraxacum mongolicum, Pollen Typhae Angustifoliae, Herba Dianthi, Homalomena occulta, Radix Peucedani, Radix Rubiae, Radix Gentianae Macrophyllae, Cortex Fraxini, Indigo Naturalis, Caulis Sinomenii, Artemisia carvifolia, Radix Aucklandiae, Pericarpium Citri Reticulatae Viride, Herba Swertiae Mileensis, Rhizoma Bistortae, Ginseng Radix et Rhizoma, Folium Ginseng, Herba cistanches, Cinnamomum cassia Presl, Saururus chinensis, Notoginseng Radix Et Rhizoma, cortex mori, Herba Taxilli, Mori Folium, Hippophae, Semen Astragali Complanati, Rhizoma Kaempferiae, Rhizoma Belamcandae, Herba Lycopodii, Cimicifugae Rhizoma, Rhizoma Acori Tatarinowii, Pericarpium Granati, Calyx Kaki, Polygoni Multiflori Caulis, Lignum Sappan, Semen Ziziphi spinosae, Herba Cynomorii, Radix Pseudstellariae, Trichosanthis Radix, Radix Semiaquilegiae, Bulbus Fritillariae Thunbergii, Radix Inulae Helenii, Santali Albi Lignum, Potentillae Chinensis Herba, Radix Linderae, Croci Stigma, Radix Panacis Quinquefolii, Spica Prunellae, Corydafis Decumbentis Rhizoma, Cyperi Rhizoma, Foeniculi Fructus, Herba Cirsii Setosi, Bulbus Allii Macrostemonis, Flos Magnoliae, Radix Cynanchi Paniculati, Flos Inulae, Herba Cissampelotis, Linum usitatissimum, Rhizoma Corydalis, Flos Chrysanthemi Indici, Fritillariae Pallidiflorae Bulbus, Leonuri Herba, Herba Epimedii, Radix Stellariae, Folium Ginkgo, Houttuynia cordata Thunb, Pruni Semen, Polygalae Radix, Rhizoma Acori Calami, Bulbus Fritillariae Thunbergii, Rhizoma Anemarrhenae, Gardeniae Fructus, Aurantii Fructus, Fructus Aurantii Immaturus, Sarcandrae Herba, Rhizoma panacis majoris, Rhizoma Panacis Japonici, Radix Amebiae, Herba Violae, Folium Perillae and Trachycarpus fortunei; and the plant extract is one or more of Armoracia rusticana powder, locust bean gum, guar gum, a black tea extract, a yellow tea extract, a white tea extract, a dark green tea extract, a black tea extract, a Fagopyrum tataricum extract, a Folium Nelumbinis extract, a Phyllostachys nigra extract, a Taxus chinensis extract, a Camptotheca acuminata extract, a Catharanthus roseus extract, a Rauvolfia verticillate extract, a Styrax benzoin extract, a Fructus Anisi Stellatim extract, a Rhizoma Cynanchi Stauntonii extract, a Radix Paeoniae Alba extract, an Atractylodes macrocephala extract, a Pulsatilla chinensis extract, a Cortex Dictamni extract, a Radix Angelicae Dahuricae extract, a Radix Isatidis extract, a Fructus Piperis Longi extract, a Herba Polygoni Avicularis extract, an Areca catechu extract, a Herba Menthaeextract, a Psoraleae Fructus extract, an Atractylodes Lancea extract, a Alpinia katsumadai Hayata extract, a Folium Aconiti Kusnezoffii extract, a Radix Bupleuri extract, a Semen Plantaginis extract, a Pericarpium Citri Reticulatae extract, a Radix Paeoniae Rubra extract, a Caulis Clematidis Armandii extract, a Radix Vladimiriae extract, a Herba Andrographitis extract, an Ailanthi Cortex extract, an Acanthopanax senticosus extract, a Paris polyphylla extract, a Radix et Rhizoma Rhei extract, a Herba Cirsii Japonici Radix Cirsii Japonici extract, a Folium Isatidis extract, a Caulis Sargentodoxae extract, a Cortex Illicii extract, a Fructus Kochiae Scopariae extract, a Radix Rehmanniae extract, a Radix Sanguisorbae extract, a Herba Belladonnae extract, a Caulis Erycibes extract, a Syringa vulgaris extract, a Malvae Fructus extract, an Alpinia katsumadai Hayata extract, an Angelicae Pubescentis Radix extract, a Cortex Eucommiae extract, a Clinopodii Herba extract, a Radix Changii extract, a Folium Sennae extract, a Radix Saposhnikoviae extract, a Radix Stephaniae Tetrandrae extract, a Rhizoma Dioscoreae Septemlobae extract, a Fructus Citri Sarcodactylis extract, a Poria cocos extract, a Rhizoma Zingiberis extract, a Radix Glycyrrhizae extract, a Rhizoma Ligustici extract, a Radix Puerariae Lobatae extract, a Ramulus Uncariae Cum Uncis extract, a Rhizoma Drynariae extract, a Pericarpium Trichosanthis extract, a Desmodium styracifolium extract, a Cinnamomi Ramulus extract, a seaweed extract, a Fructus Chebulae extract, an Albizia julibrissin extract, a Fallopia multiflora extract, a Ginseng Radix Rubra extract, a Rhodiola rosea extract, a Radix knoxiae extract, a Fructus Galangae extract, a Flos Carthami extract, a Hedysari Radix extract, a Cortex Magnoliae Officinalis extract, a Rhizoma Picrorhizae extract, a Pipemigrum extract, a Bulbus Fritillariae Hupehensis extract, a Semen Trigonellae extract, a Viscum coloratum extract, a Rhizoma Polygoni Cuspidati extract, a Zanthoxylum bungeanum Maxim extract, a Citri Grandis Exocarpium extract, a Flos Sophorae extract, a Phellodendri Chinensis Cortex extract, a Rhizoma Coptidis extract, a Radix Astragali extract, a Radix Scutellariae extract, a Fructus Cannabis extract, a Celosia cristata extract, a Herba Abri extract, a Rhizoma Curcumae Longae extract, a Lignum Dalbergiae Odoriferae extract, a Gardeniae Fructus Praeparatus extract, a Sinapis Semen extract, a Radix Tinosporae extract, a Fagopyri Dibotryis Rhizoma extract, a Lonicera JapanicaThunb extract, a Rosae Laevigatae Fructus extract, a Calyx seu Fructus Physalis extract, a Schizonepetae Herba extract, a Murraya exotica extract, a Platycodonis Radix extract, a Flos Chrysanthemi, Exocarpium Citri Rubrum extract, a Herba Selaginellae extract, a Semen Cassiae extract, a Radix Sophorae Fiavescentis extract, a Thallus Laminariae extract, a Semen Raphani extract, a Glechoma longituba extract, a Forsythia suspensa extract, a Receptaculum Nelumbinis extract, a Semen Nelumbinis extract, a Zanthoxylum nitidum extract, a Folium polygoni Tinctorll extract, a Campsis grandiflora extract, a Rhapontici Radix extract, a Rhizoma Phragmitis extract, a Fructus Momordicae extract, a Herba Ephedrae extract, a Verbena officinali extract, a Portulacae Herba extract, an Aristolochia debilis extract, a Rhododendri Daurici Folium extract, a Fructus Viticis extract, a Terminalia chebula extract, a Cortex Moutan extract, a Herba Echiptae extract, a Chaenomelis Fructus extract, a Semen Oroxyli extract, a Radix Aucklandiae extract, a Radix et Rhizoma Baphicacanthi extract, a Radix Adenophorae extract, a Fructus Schisandrae Sphenantherae extract, a Fructus Arctii extract, an Achyranthes bidentata Blume extract, a Semen Sterculiae Lychnophorae extract, a Eupatorii Herba extract, a Taraxacum mongolicum extract, a Pollen Typhae Angustifoliae extract, a Herba Dianthi extract, a Homalomena occulta extract, a Radix Peucedani extract, a Radix Rubiae extract, a Radix Gentianae Macrophyllae extract, a Cortex Fraxini extract, an Indigo Naturalis extract, a Caulis Sinomenii extract, an Artemisia carvifolia extract, a Radix Aucklandiae extract, a Pericarpium Citri Reticulatae Viride extract, a Herba Swertiae Mileensis extract, a Rhizoma Bistortae extract, a Ginseng Radix et Rhizoma extract, a Folium Ginseng extract, a Herba cistanches extract, a Cinnamomum cassia Presl extract, a Saururus chinensis extract, a Notoginseng Radix Et Rhizoma extract, a cortex mori extract, a Herba Taxilli extract, a Mori Folium extract, a Hippophae extract, a Semen Astragali Complanati extract, a Rhizoma Kaempferiae extract, a Rhizoma Belamcandae extract, a Herba Lycopodii extract, a Cimicifugae Rhizoma extract, a Rhizoma Acori Tatarinowii extract, a Pericarpium Granati extract, a Calyx Kaki extract, a Polygoni Multiflori Caulis extract, a Lignum Sappan extract, a Semen Ziziphi spinosae extract, a Herba Cynomorii extract, a Radix Pseudstellariae extract, a Trichosanthis Radix extract, a Radix Semiaquilegiae extract, a Bulbus Fritillariae Thunbergii extract, a Radix Inulae Helenii extract, a Santali Albi Lignum extract, a Potentillae Chinensis Herba extract, a Radix Linderae extract, a Croci Stigma extract, a Radix Panacis Quinquefolii extract, a Spica Prunellae extract, a Corydalis Decumbentis Rhizoma extract, a Cyperi Rhizoma extract, a Foeniculi Fructus extract, a Herba Cirsii Setosi extract, a Bulbus Allii Macrostemonis extract, a Flos Magnoliae extract, a Radix Cynanchi Paniculati extract, a Flos Inulae extract, a Herba Cissampelotis extract, a Linum usitatissimum, Rhizoma Corydalis extract, a Flos Chrysanthemi Indici extract, a Fritillariae Pallidiflorae Bulbus extract, a Leonuri Herba extract, a Herba Epimedii extract, a Radix Stellariae extract, a Folium Ginkgo extract, a Houttuynia cordata Thunb extract, a Pruni Semen extract, a Polygalae Radix extract, a Rhizoma Acori Calami extract, a Bulbus Fritillariae Thunbergii extract, a Rhizoma Anemarrhenae extract, a Gardeniae Fructus extract, an Aurantii Fructus extract, a Fructus Aurantii Immaturus extract, a Sarcandrae Herba extract, a Rhizoma panacis majoris extract, a Rhizoma Panacis Japonici extract, a Radix Amebiae extract, a Herba Violae extract, a Folium Perillae extract and a Trachycarpus fortune extract.

The food-induced and/or drug-induced plants and their extracts that can be used as password components of the mycelium packaging material include, but are not limited to, all of the above-mentioned materials, and all plants and their extracts with conditions and characteristics to be identified are included in the scope of patent protection of the present application.

The basidiomycete strain is one of oyster mushroom, pleurotus nebrodensis, Ganoderma lucidum, shiitake mushroom, straw mushroom, enoki mushroom and trametes gibbosa, and has an inoculation amount of 10-30% of the total weight of the biological raw material. The wood fiber is one or more of hemp stalks, wheat straws, corn straws, miscellaneous sawdust, and soybean straws; the wood fiber has a particle size of 0-1 cm (excluding an endpoint value 0); and the wood fiber consists of 15-50 parts by weight of hemp stalks, 0-15 parts by weight of wheat straws, 15-40 parts by weight of miscellaneous sawdust, 0-35 parts by weight of corn straws, and 0-20 parts by weight of soybean straws.

### Example 2

A mycelium packaging material having an anti-counterfeiting function is different from Example 1 in that: the addition amount of the password component is 0.5-5% of the total weight of the biological raw material.

### Example 3

A method for using the mycelium packaging material having the anti-counterfeiting function includes the following steps which are implemented in sequence:
S1: preparation of culture material: first preparing a wood fiber and an auxiliary component according to a formula, mixing well with water, and stacking and fermenting for 12-24 h; then adding a nutritional component to prepare a biological raw material, mixing well, and autoclaving for 1-2 h under the conditions that the temperature is 121-126°C and the pressure is 0.12-0.15 MPa; then inoculating a basidiomycete strain; and finally mixing a password component into the inoculated biological raw material, and mixing well to obtain the culture material;
S2: molding culture: putting the culture material and a subject matter together in a breathable and waterproof sterile mold such that the culture material evenly wraps an outer wall of the subject matter; putting a filler block on the outer wall of the subject matter corresponding to a trademark position; and finally culturing in the dark for 5-8 days under the conditions that the temperature is 20-30°C and the humidity is 90-100%, taking the filler block away, and air-drying in natural air until the water content of the mycelium packaging material having the anti-counterfeiting function is less than 15%, and the anti-counterfeiting subject matter is obtained; and
S3: anti-counterfeiting detection: first observing whether a material around a suspected subject matter is smooth and flat, whether the color is uniform, whether a surface mycelium is white and dense, and whether the structure is intact; then detecting physical properties of the material around the suspected subject matter; and finally taking the material around the suspected subject matter (an amount of the taken material is related to the addition amount of the password component and an amount required in its detection method), selecting a corresponding detection method according to the password component added in the corresponding batch, i.e., selecting a corresponding detection method according to specific active ingredients contained in food-induced and/or drug-induced plants, and food-induced and/or drug-induced plant extracts, and detecting whether the material contains a specific password component, e.g., detecting whether the material contains the added password component according to the corresponding detection methods of different Chinese herbal materials recorded in the Chinese Pharmacopoeia. The password component should be ensured to be sterile before adding, and needs to be sterilized if the microbial content does not meet requirements, and an appropriate sterilization method is selected according to the characteristics of the password component itself.

According to the method in Example 3, the subject matter is packaged, and an appearance effect diagram of the obtained anti-counterfeiting subject object was shown in detail in FIG. 1. As can be seen from FIG. 1, the sample subject matter was completely wrapped by the surrounding material, the trademark of the subject matter was clearly exposed, the surrounding material of the subject matter was smooth and flat and was uniform in color, the surface mycelium was white and dense, and the structure was intact.

In order to further illustrate the beneficial effects of the present application, the following is further illustrated according to the specific test examples and comparative examples.

### Test example 1

A method for using the mycelium packaging material having the anti-counterfeiting function includes the following steps which are implemented in sequence:
S1: preparation of culture material: first mixing 45 parts by weight of hemp stalks, 15 parts by weight of miscellaneous sawdust, 15 parts by weight of corn stalks, 5 parts by weight of wheat straws, 2 parts by weight of gypsum powder, and 2 parts by weight of quicklime and adding water, wherein the addition amount of water is 1.4 times the total weight of the wood fiber, the auxiliary component and the nutritional component; adjusting a pH value of the mixture to 7.0 with quicklime, mixing, and then stacking and fermenting for 18 h; then adding 10 parts by weight of wheat bran and 10 parts by weight of corn starch to obtain a biological raw material; after mixing well, autoclaving for 2 h under the conditions that the temperature is 121°C and the pressure is 0.15 MPa; then inoculating a Ganoderma lucidum strain at an amount of 20% of the total weight of the biological raw material; and finally, mixing Flos Sophorae (crushed to a particle size of about 0.5 cm) into the inoculated biological raw material according to an amount of 2% of the total weight of the biological raw material, and mixing well to obtain the culture material;
S2: molding culture: putting the culture material and a subject matter together in a breathable and waterproof square sterile mold of 34 cm×12 cm×9 cm such that the culture material evenly wraps an outer wall of the subject matter; putting a filler block on an outer wall of the subject matter corresponding to a trademark position; and finally culturing in the dark for 6 days under the conditions that the temperature is 25°C and the humidity is 95%, taking the filler block away to expose the trademark of the subject matter, and air-drying in natural air until the water content of the mycelium packaging material having the anti-counterfeiting function is less than 15%, and the anti-counterfeiting subject matter is obtained; and
S3: anti-counterfeiting detection: detecting whether the material of the subject matter in this test example is smooth and flat, whether the color is uniform, whether the surface mycelium is white and dense, and whether the structure is intact without damage; according to the national standards GB/T 8813-2008 and GB/T 8812-2007, measuring a compressive strength and bending strength of the product, wherein the compressive strength is 152.13 kPa, and the bending strength is 166.32 kPa; taking 10 g of the material around the subject matter, crushing, adding 20 ml of ethanol, heating for 5 minutes, and filtering; taking 1 ml of filtrate, adding a small amount of magnesium powder and 2-3 drops of hydrochloric acid, and determining that a password component of Flos Sophorae is included if the color is cherry red; or by taking rutin as a control, detecting whether the password component exists according to the thin layer chromatography test, wherein the result showed that Flos Sophorae is detected.

### Test example 2

A method for using the mycelium packaging material having the anti-counterfeiting function includes the following steps which are implemented in sequence:
S1: preparation of culture material: first mixing 15 parts by weight of hemp stalks, 25 parts by weight of miscellaneous sawdust, 10 parts of soybean straws, 10 parts by weight of corn stalks, 3 parts by weight of gypsum powder, and 2 parts by weight of quicklime and adding water, wherein the addition amount of water is 1.3 times the total weight of the wood fiber, the auxiliary component and the nutritional component; adjusting a pH value of the mixture to 7.0 with quicklime, mixing, and then stacking and fermenting for 12 h; then adding 20 parts by weight of wheat bran and 5 parts by weight of corn starch to obtain a biological raw material; after mixing well, autoclaving for 1.5 h under the conditions that the temperature is 121°C and the pressure is 0.15 MPa; then inoculating a pleurotus nebrodensis strain at an amount of 25% of the total weight of the biological raw material; and finally, mixing Armoracia rusticana powder into the inoculated biological raw material according to an amount of 0.5% of the total weight of the biological raw material, and mixing well to obtain the culture material;
S2: molding culture: putting the culture material and a subject matter together in a breathable and waterproof square sterile mold of 34 cm×12 cm×9 cm such that the culture material evenly wraps an outer wall of the subject matter; putting a filler block on an outer wall of the subject matter corresponding to a trademark position; and finally culturing in the dark for 8 days under the conditions that the temperature is 24°C and the humidity is 98%, taking the filler block away to expose the trademark of the subject matter, and air-drying in natural air until the water content of the mycelium packaging material having the anti-counterfeiting function is less than 15%, and the anti-counterfeiting subject matter is obtained; and
S3: anti-counterfeiting detection: detecting whether the material of the subject matter in this test example is smooth and flat, whether the color is uniform, whether the surface mycelium is white and dense, and whether the structure is intact without damage; according to the national standards GB/T 8813-2008 and GB/T 8812-2007, measuring a compressive strength and bending strength of the product, wherein the compressive strength is 142.53 kPa, and the bending strength is 136.28 kPa; taking the material around the subject matter, and according to the detection method recorded in the Chinese Pharmacopoeia, detecting the content of sinapine by HPLC to determine whether a password component of Armoracia rusticana powder exists, wherein the results showed that Armoracia rusticana is detected.

### Test example 3

A method for using the mycelium packaging material having the anti-counterfeiting function includes the following steps which are implemented in sequence:
S1: preparation of culture material: first mixing 40 parts by weight of hemp stalks, 20 parts by weight of corn stalks, 15 parts by weight of miscellaneous sawdust, 2 parts by weight of gypsum powder, and 1 part by weight of quicklime and adding water, wherein the addition amount of water is 1.3 times the total weight of the wood fiber, the auxiliary component and the nutritional component; adjusting a pH value of the mixture to 7.0 with quicklime, mixing, and then stacking and fermenting for 24 h; then adding 15 parts by weight of wheat bran and 10 parts by weight of corn starch to obtain a biological raw material; after mixing well, autoclaving for 2 h under the conditions that the temperature is 121°C and the pressure is 0.15 MPa; then inoculating a straw mushroom strain at an amount of 30% of the total weight of the biological raw material; and finally, mixing green tea having a particle size of 0-0.6 cm into the inoculated biological raw material according to an amount of 5% of the total weight of the biological raw material, and mixing well to obtain the culture material;
S2: molding culture: putting the culture material and a subject matter together in a breathable and waterproof square sterile mold of 34 cm×12 cm×9 cm such that the culture material evenly wraps an outer wall of the subject matter; putting a filler block on the outer wall of the subject matter corresponding to a trademark position; and finally culturing in the dark for 7 days under the conditions that the temperature is 30°C and the humidity is 90%, taking the filler block away to expose the trademark of the subject matter, and air-drying in natural air until the water content of the mycelium packaging material having the anti-counterfeiting function is less than 15%, and the anti-counterfeiting subject matter is obtained; and
S3: anti-counterfeiting detection: detecting whether the material of the subject matter in this test example is smooth and flat, whether the color is uniform, whether the surface mycelium is white and dense, and whether the structure is intact without damage; according to the national standards GB/T 8813-2008 and GB/T 8812-2007, measuring a compressive strength and bending strength of the product, wherein the compressive strength is 158.82 kPa, and the bending strength is 146.87 kPa; taking the material around the subject matter, and by taking the green tea extract (tea polyphenol) as a control, detecting whether a password component exists by HPLC, wherein the results show that tea polyphenol is detected.

In the above examples and test examples, stacking fermentation functions to kill some microorganisms first, and then make wood fiber components fully absorb water, which is conducive to thorough subsequent autoclaving and mycelium growth. Because nutrients are added before fermentation, it is easy for microorganisms to breed and cause rancidity of the materials. Therefore, in order to prevent rancidity of the raw materials, nutrients are added after fermentation. The water was added in a required amount according to a planned total consumption of the wood fiber, auxiliary component and nutritional component.

### Comparative example 1

A method for using the mycelium packaging material having the anti-counterfeiting function includes the following steps which are implemented in sequence:
S1: preparation of culture material: first mixing 10 parts by weight of miscellaneous sawdust, 20 parts by weight of hemp stalks, 45 parts by weight of corn stalks, 3 parts by weight of gypsum powder, and 3 parts by weight of quicklime and adding water, wherein the addition amount of water is 1.35 times the total weight of the wood fiber, the auxiliary component and the nutritional component; adjusting a pH value of the mixture to 7.0 with quicklime, mixing, and then stacking and fermenting for 24 h; then adding 5 parts by weight of wheat bran and 15 parts by weight of corn starch to obtain a biological raw material; after mixing well, autoclaving for 2 h under the conditions that the temperature is 121°C and the pressure is 0.15 MPa; then inoculating a Ganoderma lucidum strain at an amount of 30% of the total weight of the biological raw material to obtain the culture material;
S2: molding culture: putting the culture material and a subject matter together in a waterproof square sterile mold of 34 cm×12 cm×9 cm such that the culture material evenly wraps an outer wall of the subject matter; putting a filler block on the outer wall of the subject matter corresponding to a trademark position; and finally culturing in the dark for 6 days under the conditions that the temperature is 25°C and the humidity is 95%, taking the filler block away to expose the trademark of the subject matter, and air-drying in natural air until the water content of the mycelium packaging material having the anti-counterfeiting function is less than 15%, and the anti-counterfeiting subject matter is obtained; and
S3: anti-counterfeiting detection: detecting whether the material of the subject matter in this comparative example is smooth and flat, whether the color is uniform, whether the raw material is exposed, whether the surface mycelium is sparse and slender, and whether the structure is intact; according to the national standards GB/T 8813-2008 and GB/T 8812-2007, measuring a compressive strength and bending strength of the product, wherein the compressive strength is 92.43 kPa, and the bending strength is 88.56 kPa; taking the material around the subject matter, and detecting in accordance with the detection methods recorded in the Chinese Pharmacopoeia (if not recorded in the Chinese Pharmacopoeia, the existing technical methods such as HPLC are directly used), wherein characteristic ingredients of food-induced and drug-induced plants such as green tea, black tea, yellow tea, white tea, dark green tea, black tea, Fagopyrum tataricum, Folium Nelumbinis, Phyllostachys nigra, Taxus chinensis, Armoracia rusticana, Camptotheca acuminata, Catharanthus roseus, Rauvolfia verticillata, Styrax benzoin, Fructus Anisi Stellatim, Rhizoma Cynanchi Stauntonii, Radix Paeoniae Alba, Atractylodes macrocephala, Pulsatilla chinensis, Cortex Dictamni, Radix Angelicae Dahuricae, Radix Isatidis, Fructus Piperis Longi, Herba Polygoni Avicularis, Areca catech, Herba Menthae, Psoraleae Fructus, Atractylodes Lancea, Alpinia katsumadai Hayata , Folium Aconiti Kusnezoffii, Radix Bupleuri, Semen Plantaginis, Pericarpium Citri Reticulatae, Radix Paeoniae Rubra, Caulis Clematidis Armandii, Radix Vladimiriae, Herba Andrographitis, Ailanthi Cortex, Acanthopanax senticosus, Paris polyphylla, Radix et Rhizoma Rhei, Herba Cirsii Japonici Radix Cirsii Japonici, Folium Isatidis, Caulis Sargentodoxae, Cortex Illicii, Fructus Kochiae Scopariae, Radix Rehmanniae, Radix Sanguisorbae, Herba Belladonnae, Caulis Erycibes, Syringa vulgaris, Malvae Fructus, Alpinia katsumadai Hayata, Angelicae Pubescentis Radix, Cortex Eucommiae, Clinopodii Herba, Radix Changii, Folium Sennae, Radix Saposhnikoviae, Radix Stephaniae Tetrandrae, Rhizoma Dioscoreae Septemlobae, Fructus Citri Sarcodactylis, Poria cocos, Rhizoma Zingiberis, Radix Glycyrrhizae, Rhizoma Ligustici, Radix Puerariae Lobatae, Ramulus Uncariae Cum Uncis, Rhizoma Drynariae, Pericarpium Trichosanthis, Desmodium styracifolium, Cinnamomi Ramulus, seaweed, Fructus Chebulae, Albizia julibrissin, Fallopia multiflora, Ginseng Radix Rubra, Rhodiola rosea, Radix knoxiae, Fructus Galangae, Flos Carthami, Hedysari Radix, Cortex Magnoliae Officinalis, Rhizoma Picrorhizae, Pipemigrum, Bulbus Fritillariae Hupehensis, Semen Trigonellae, Viscum coloratum, Rhizoma Polygoni Cuspidati, Zanthoxylum bungeanum Maxim, Citri Grandis Exocarpium, Flos Sophorae, Phellodendri Chinensis Cortex, Rhizoma Coptidis, Radix Astragali, Radix Scutellariae, Fructus Cannabis, Celosia cristata, Herba Abri, Rhizoma Curcumae Longae, Lignum Dalbergiae Odoriferae, Gardeniae Fructus Praeparatus, Sinapis Semen, Radix Tinosporae, Fagopyri Dibotryis Rhizoma, Lonicera JapanicaThunb, Rosae Laevigatae Fructus, Calyx seu Fructus Physalis, Schizonepetae Herba, Murraya exotica, Platycodonis Radix, Flos Chrysanthemi, Exocarpium Citri Rubrum, Herba Selaginellae, Semen Cassiae, Radix Sophorae Fiavescentis, Thallus Laminariae, Semen Raphani, Glechoma longituba, Forsythia suspensa, Receptaculum Nelumbinis, Semen Nelumbinis, Zanthoxylum nitidum, Folium polygoni Tinctorll, Campsis grandiflora, Rhapontici Radix, Rhizoma Phragmitis, Fructus Momordicae, Herba Ephedrae, Verbena officinali, Portulacae Herba, Aristolochia debilis, Rhododendri Daurici Folium, Fructus Viticis, Terminalia chebula, Cortex Moutan, Herba Echiptae, Chaenomelis Fructus, Semen Oroxyli, Radix Aucklandiae, Radix et Rhizoma Baphicacanthi, Radix Adenophorae, Fructus Schisandrae Sphenantherae, Fructus Arctii, Achyranthes bidentata Blume, Semen Sterculiae Lychnophorae, Eupatorii Herba, Taraxacum mongolicum, Pollen Typhae Angustifoliae, Herba Dianthi, Homalomena occulta, Radix Peucedani, Radix Rubiae, Radix Gentianae Macrophyllae, Cortex Fraxini, Indigo Naturalis, Caulis Sinomenii, Artemisia carvifolia, Radix Aucklandiae, Pericarpium Citri Reticulatae Viride, Herba Swertiae Mileensis, Rhizoma Bistortae, Ginseng Radix et Rhizoma, Folium Ginseng, Herba cistanches, Cinnamomum cassia Presl, Saururus chinensis, Notoginseng Radix Et Rhizoma, cortex mori, Herba Taxilli, Mori Folium, Hippophae, Semen Astragali Complanati, Rhizoma Kaempferiae, Rhizoma Belamcandae, Herba Lycopodii, Cimicifugae Rhizoma, Rhizoma Acori Tatarinowii, Pericarpium Granati, Calyx Kaki, Polygoni Multiflori Caulis, Lignum Sappan, Semen Ziziphi spinosae, Herba Cynomorii, Radix Pseudstellariae, Trichosanthis Radix, Radix Semiaquilegiae, Bulbus Fritillariae Thunbergii, Radix Inulae Helenii, Santali Albi Lignum, Potentillae Chinensis Herba, Radix Linderae, Croci Stigma, Radix Panacis Quinquefolii, Spica Prunellae, Corydalis Decumbentis Rhizoma, Cyperi Rhizoma, Foeniculi Fructus, Herba Cirsii Setosi, Bulbus Allii Macrostemonis, Flos Magnoliae, Radix Cynanchi Paniculati, Flos Inulae, Herba Cissampelotis, Linum usitatissimum, Rhizoma Corydalis, Flos Chrysanthemi Indici, Fritillariae Pallidiflorae Bulbus, Leonuri Herba, Herba Epimedii, Radix Stellariae, Folium Ginkgo, Houttuynia cordata Thunb, Pruni Semen, Polygalae Radix, Rhizoma Acori Calami, Bulbus Fritillariae Thunbergii, Rhizoma Anemarrhenae, Gardeniae Fructus, Aurantii Fructus, Fructus Aurantii Immaturus, Sarcandrae Herba, Rhizoma panacis majoris, Rhizoma Panacis Japonici, Radix Amebiae, Herba Violae, Folium Perillae and Trachycarpus fortunei, extracts thereof, as well as Armoracia rusticana powder, locust bean gum, guar gum, etc. are not detected.

### Comparative example 2

A method for using the mycelium packaging material having the anti-counterfeiting function includes the following steps which are implemented in sequence:
S1: preparation of culture material: first mixing 60 parts by weight of miscellaneous sawdust, 10 parts by weight of hemp stalks, 20 parts by weight of wheat stalks, 2 parts by weight of gypsum powder, and 3 parts by weight of quicklime and adding water, wherein the addition amount of water is 1.35 times the total weight of the wood fiber, the auxiliary component and the nutritional component; adjusting a pH value of the mixture to 7.0 with quicklime, mixing, and then stacking and fermenting for 18 h; then adding 20 parts by weight of wheat bran and 5 parts by weight of corn starch to obtain a biological raw material; after mixing well, autoclaving for 2 h under the conditions that the temperature is 121°C and the pressure is 0.15 MPa; then inoculating a trametes gibbosa strain at an amount of 20% of the total weight of the biological raw material to obtain the culture material;
S2: molding culture: putting the culture material and a subject matter together in a breathable and waterproof square sterile mold of 40 cm×20 cm×7.5 cm such that the culture material evenly wraps an outer wall of the subject matter; putting a filler block on the outer wall of the subject matter corresponding to a trademark position; and finally culturing in the dark for 8 days under the conditions that the temperature is 24°C and the humidity is 96%, taking the filler block away to expose the trademark of the subject matter, and air-drying in natural air until the water content of the mycelium packaging material having the anti-counterfeiting function is less than 15%, and the anti-counterfeiting subject matter is obtained; and
S3: anti-counterfeiting detection: detecting whether the material of the subject matter in this comparative example is unsmooth and flat, whether the color is non-uniform, and whether there are patches of macula (patches of macula are caused by overgrowth of aerial hyphae at a macula location); according to the national standards GB/T 8813-2008 and GB/T 8812-2007, measuring a compressive strength and bending strength of the product, wherein the compressive strength is 86.22 kPa, and the bending strength is 92.16 kPa; taking the material around the subject matter, and detecting in accordance with the detection methods recorded in the Chinese Pharmacopoeia (if not recorded in the Chinese Pharmacopoeia, the existing technical methods such as HPLC are directly used), wherein characteristic ingredients of food-induced and drug-induced plants such as green tea, black tea, yellow tea, white tea, dark green tea, black tea, Fagopyrum tataricum, Folium Nelumbinis, Phyllostachys nigra, Taxus chinensis, Armoracia rusticana, Camptotheca acuminata, Catharanthus roseus, Rauvolfia verticillata, Styrax benzoin, Fructus Anisi Stellatim, Rhizoma Cynanchi Stauntonii, Radix Paeoniae Alba, Atractylodes macrocephala, Pulsatilla chinensis, Cortex Dictamni, Radix Angelicae Dahuricae, Radix Isatidis, Fructus Piperis Longi, Herba Polygoni Avicularis, Areca catech, Herba Menthae, Psoraleae Fructus, Atractylodes Lancea, Alpinia katsumadai Hayata , Folium Aconiti Kusnezoffii, Radix Bupleuri, Semen Plantaginis, Pericarpium Citri Reticulatae, Radix Paeoniae Rubra, Caulis Clematidis Armandii, Radix Vladimiriae, Herba Andrographitis, Ailanthi Cortex, Acanthopanax senticosus, Paris polyphylla, Radix et Rhizoma Rhei, Herba Cirsii Japonici Radix Cirsii Japonici, Folium Isatidis, Caulis Sargentodoxae, Cortex Illicii, Fructus Kochiae Scopariae, Radix Rehmanniae, Radix Sanguisorbae, Herba Belladonnae, Caulis Erycibes, Syringa vulgaris, Malvae Fructus, Alpinia katsumadai Hayata, Angelicae Pubescentis Radix, Cortex Eucommiae, Clinopodii Herba, Radix Changii, Folium Sennae, Radix Saposhnikoviae, Radix Stephaniae Tetrandrae, Rhizoma Dioscoreae Septemlobae, Fructus Citri Sarcodactylis, Poria cocos, Rhizoma Zingiberis, Radix Glycyrrhizae, Rhizoma Ligustici, Radix Puerariae Lobatae, Ramulus Uncariae Cum Uncis, Rhizoma Drynariae, Pericarpium Trichosanthis, Desmodium styracifolium, Cinnamomi Ramulus, seaweed, Fructus Chebulae, Albizia julibrissin, Fallopia multiflora, Ginseng Radix Rubra, Rhodiola rosea, Radix knoxiae, Fructus Galangae, Flos Carthami, Hedysari Radix, Cortex Magnoliae Officinalis, Rhizoma Picrorhizae, Pipemigrum, Bulbus Fritillariae Hupehensis, Semen Trigonellae, Viscum coloratum, Rhizoma Polygoni Cuspidati, Zanthoxylum bungeanum Maxim, Citri Grandis Exocarpium, Flos Sophorae, Phellodendri Chinensis Cortex, Rhizoma Coptidis, Radix Astragali, Radix Scutellariae, Fructus Cannabis, Celosia cristata, Herba Abri, Rhizoma Curcumae Longae, Lignum Dalbergiae Odoriferae, Gardeniae Fructus Praeparatus, Sinapis Semen, Radix Tinosporae, Fagopyri Dibotryis Rhizoma, Lonicera JapanicaThunb, Rosae Laevigatae Fructus, Calyx seu Fructus Physalis, Schizonepetae Herba, Murraya exotica, Platycodonis Radix, Flos Chrysanthemi, Exocarpium Citri Rubrum, Herba Selaginellae, Semen Cassiae, Radix Sophorae Fiavescentis, Thallus Laminariae, Semen Raphani, Glechoma longituba, Forsythia suspensa, Receptaculum Nelumbinis, Semen Nelumbinis, Zanthoxylum nitidum, Folium polygoni Tinctorll, Campsis grandiflora, Rhapontici Radix, Rhizoma Phragmitis, Fructus Momordicae, Herba Ephedrae, Verbena officinali, Portulacae Herba, Aristolochia debilis, Rhododendri Daurici Folium, Fructus Viticis, Terminalia chebula, Cortex Moutan, Herba Echiptae, Chaenomelis Fructus, Semen Oroxyli, Radix Aucklandiae, Radix et Rhizoma Baphicacanthi, Radix Adenophorae, Fructus Schisandrae Sphenantherae, Fructus Arctii, Achyranthes bidentata Blume, Semen Sterculiae Lychnophorae, Eupatorii Herba, Taraxacum mongolicum, Pollen Typhae Angustifoliae, Herba Dianthi, Homalomena occulta, Radix Peucedani, Radix Rubiae, Radix Gentianae Macrophyllae, Cortex Fraxini, Indigo Naturalis, Caulis Sinomenii, Artemisia carvifolia, Radix Aucklandiae, Pericarpium Citri Reticulatae Viride, Herba Swertiae Mileensis, Rhizoma Bistortae, Ginseng Radix et Rhizoma, Folium Ginseng, Herba cistanches, Cinnamomum cassia Presl, Saururus chinensis, Notoginseng Radix Et Rhizoma, cortex mori, Herba Taxilli, Mori Folium, Hippophae, Semen Astragali Complanati, Rhizoma Kaempferiae, Rhizoma Belamcandae, Herba Lycopodii, Cimicifugae Rhizoma, Rhizoma Acori Tatarinowii, Pericarpium Granati, Calyx Kaki, Polygoni Multiflori Caulis, Lignum Sappan, Semen Ziziphi spinosae, Herba Cynomorii, Radix Pseudstellariae, Trichosanthis Radix, Radix Semiaquilegiae, Bulbus Fritillariae Thunbergii, Radix Inulae Helenii, Santali Albi Lignum, Potentillae Chinensis Herba, Radix Linderae, Croci Stigma, Radix Panacis Quinquefolii, Spica Prunellae, Corydalis Decumbentis Rhizoma, Cyperi Rhizoma, Foeniculi Fructus, Herba Cirsii Setosi, Bulbus Allii Macrostemonis, Flos Magnoliae, Radix Cynanchi Paniculati, Flos Inulae, Herba Cissampelotis, Linum usitatissimum, Rhizoma Corydalis, Flos Chrysanthemi Indici, Fritillariae Pallidiflorae Bulbus, Leonuri Herba, Herba Epimedii, Radix Stellariae, Folium Ginkgo, Houttuynia cordata Thunb, Pruni Semen, Polygalae Radix, Rhizoma Acori Calami, Bulbus Fritillariae Thunbergii, Rhizoma Anemarrhenae, Gardeniae Fructus, Aurantii Fructus, Fructus Aurantii Immaturus, Sarcandrae Herba, Rhizoma panacis majoris, Rhizoma Panacis Japonici, Radix Amebiae, Herba Violae, Folium Perillae and Trachycarpus fortunei, extracts thereof, as well as Armoracia rusticana powder, locust bean gum, guar gum, etc. are not detected.

### Comparative example 3

A method for using the mycelium packaging material having the anti-counterfeiting function includes the following steps which are implemented in sequence:
S1: preparation of culture material: first mixing 30 parts by weight of hemp stalks, 25 parts by weight of corn stalks, 15 parts by weight of miscellaneous sawdust, 15 parts by weight of white stalks, 2 parts by weight of gypsum powder, and 3 parts by weight of quicklime and adding water, wherein the addition amount of water is 1.3 times the total weight of the wood fiber, the auxiliary component and the nutritional component; adjusting a pH value of the mixture to 7.0 with quicklime, mixing, and then stacking and fermenting for 24 h; then adding 20 parts by weight of wheat bran and 10 parts by weight of corn starch to obtain a biological raw material; after mixing well, autoclaving for 2 h under the conditions that the temperature is 121°C and the pressure is 0.15 MPa; then inoculating a Ganoderma lucidum strain at an amount of 20% of the total weight of the biological raw material to obtain the culture material;
S2: molding culture: putting the culture material and a subject matter together in a breathable and waterproof square sterile mold of 40 cm×20 cm×7.5 cm such that the culture material evenly wraps an outer wall of the subject matter; putting a filler block on an outer wall of the subject matter corresponding to a trademark position; and finally culturing in the dark for 7 days under the conditions that the temperature is 25°C and the humidity is 95%, taking the filler block away to expose the trademark of the subject matter, and air-drying in natural air until the water content of the mycelium packaging material having the anti-counterfeiting function is less than 15%, and the anti-counterfeiting subject matter is obtained; and
S3: anti-counterfeiting detection: detecting whether the material of the subject matter in this test example is smooth and flat, whether the color is uniform, whether the surface mycelium are white and dense, and whether the structure is intact; according to the national standards GB/T 8813-2008 and GB/T 8812-2007, measuring a compressive strength and bending strength of the product, wherein the compressive strength is 150.24 kPa, and the bending strength is 158-18 kPa; taking the material around the subject matter, and detecting in accordance with the detection methods recorded in the Chinese Pharmacopoeia (if not recorded in the Chinese Pharmacopoeia, the existing technical methods such as HPLC are directly used), wherein characteristic ingredients of food-induced and drug-induced plants such as green tea, black tea, yellow tea, white tea, dark green tea, black tea, Fagopyrum tataricum, Folium Nelumbinis, Phyllostachys nigra, Taxus chinensis, Armoracia rusticana, Camptotheca acuminata, Catharanthus roseus, Rauvolfia verticillata, Styrax benzoin, Fructus Anisi Stellatim, Rhizoma Cynanchi Stauntonii, Radix Paeoniae Alba, Atractylodes macrocephala, Pulsatilla chinensis, Cortex Dictamni, Radix Angelicae Dahuricae, Radix Isatidis, Fructus Piperis Longi, Herba Polygoni Avicularis, Areca catech, Herba Menthae, Psoraleae Fructus, Atractylodes Lancea, Alpinia katsumadai Hayata , Folium Aconiti Kusnezoffii, Radix Bupleuri, Semen Plantaginis, Pericarpium Citri Reticulatae, Radix Paeoniae Rubra, Caulis Clematidis Armandii, Radix Vladimiriae, Herba Andrographitis, Ailanthi Cortex, Acanthopanax senticosus, Paris polyphylla, Radix et Rhizoma Rhei, Herba Cirsii Japonici Radix Cirsii Japonici, Folium Isatidis, Caulis Sargentodoxae, Cortex Illicii, Fructus Kochiae Scopariae, Radix Rehmanniae, Radix Sanguisorbae, Herba Belladonnae, Caulis Erycibes, Syringa vulgaris, Malvae Fructus, Alpinia katsumadai Hayata, Angelicae Pubescentis Radix, Cortex Eucommiae, Clinopodii Herba, Radix Changii, Folium Sennae, Radix Saposhnikoviae, Radix Stephaniae Tetrandrae, Rhizoma Dioscoreae Septemlobae, Fructus Citri Sarcodactylis, Poria cocos, Rhizoma Zingiberis, Radix Glycyrrhizae, Rhizoma Ligustici, Radix Puerariae Lobatae, Ramulus Uncariae Cum Uncis, Rhizoma Drynariae, Pericarpium Trichosanthis, Desmodium styracifolium, Cinnamomi Ramulus, seaweed, Fructus Chebulae, Albizia julibrissin, Fallopia multiflora, Ginseng Radix Rubra, Rhodiola rosea, Radix knoxiae, Fructus Galangae, Flos Carthami, Hedysari Radix, Cortex Magnoliae Officinalis, Rhizoma Picrorhizae, Pipemigrum, Bulbus Fritillariae Hupehensis, Semen Trigonellae, Viscum coloratum, Rhizoma Polygoni Cuspidati, Zanthoxylum bungeanum Maxim, Citri Grandis Exocarpium, Flos Sophorae, Phellodendri Chinensis Cortex, Rhizoma Coptidis, Radix Astragali, Radix Scutellariae, Fructus Cannabis, Celosia cristata, Herba Abri, Rhizoma Curcumae Longae, Lignum Dalbergiae Odoriferae, Gardeniae Fructus Praeparatus, Sinapis Semen, Radix Tinosporae, Fagopyri Dibotryis Rhizoma, Lonicera JapanicaThunb, Rosae Laevigatae Fructus, Calyx seu Fructus Physalis, Schizonepetae Herba, Murraya exotica, Platycodonis Radix, Flos Chrysanthemi, Exocarpium Citri Rubrum, Herba Selaginellae, Semen Cassiae, Radix Sophorae Fiavescentis, Thallus Laminariae, Semen Raphani, Glechoma longituba, Forsythia suspensa, Receptaculum Nelumbinis, Semen Nelumbinis, Zanthoxylum nitidum, Folium polygoni Tinctorll, Campsis grandiflora, Rhapontici Radix, Rhizoma Phragmitis, Fructus Momordicae, Herba Ephedrae, Verbena officinali, Portulacae Herba, Aristolochia debilis, Rhododendri Daurici Folium, Fructus Viticis, Terminalia chebula, Cortex Moutan, Herba Echiptae, Chaenomelis Fructus, Semen Oroxyli, Radix Aucklandiae, Radix et Rhizoma Baphicacanthi, Radix Adenophorae, Fructus Schisandrae Sphenantherae, Fructus Arctii, Achyranthes bidentata Blume, Semen Sterculiae Lychnophorae, Eupatorii Herba, Taraxacum mongolicum, Pollen Typhae Angustifoliae, Herba Dianthi, Homalomena occulta, Radix Peucedani, Radix Rubiae, Radix Gentianae Macrophyllae, Cortex Fraxini, Indigo Naturalis, Caulis Sinomenii, Artemisia carvifolia, Radix Aucklandiae, Pericarpium Citri Reticulatae Viride, Herba Swertiae Mileensis, Rhizoma Bistortae, Ginseng Radix et Rhizoma, Folium Ginseng, Herba cistanches, Cinnamomum cassia Presl, Saururus chinensis, Notoginseng Radix Et Rhizoma, cortex mori, Herba Taxilli, Mori Folium, Hippophae, Semen Astragali Complanati, Rhizoma Kaempferiae, Rhizoma Belamcandae, Herba Lycopodii, Cimicifugae Rhizoma, Rhizoma Acori Tatarinowii, Pericarpium Granati, Calyx Kaki, Polygoni Multiflori Caulis, Lignum Sappan, Semen Ziziphi spinosae, Herba Cynomorii, Radix Pseudstellariae, Trichosanthis Radix, Radix Semiaquilegiae, Bulbus Fritillariae Thunbergii, Radix Inulae Helenii, Santali Albi Lignum, Potentillae Chinensis Herba, Radix Linderae, Croci Stigma, Radix Panacis Quinquefolii, Spica Prunellae, Corydafis Decumbentis Rhizoma, Cyperi Rhizoma, Foeniculi Fructus, Herba Cirsii Setosi, Bulbus Allii Macrostemonis, Flos Magnoliae, Radix Cynanchi Paniculati, Flos Inulae, Herba Cissampelotis, Linum usitatissimum, Rhizoma Corydalis, Flos Chrysanthemi Indici, Fritillariae Pallidiflorae Bulbus, Leonuri Herba, Herba Epimedii, Radix Stellariae, Folium Ginkgo, Houttuynia cordata Thunb, Pruni Semen, Polygalae Radix, Rhizoma Acori Calami, Bulbus Fritillariae Thunbergii, Rhizoma Anemarrhenae, Gardeniae Fructus, Aurantii Fructus, Fructus Aurantii Immaturus, Sarcandrae Herba, Rhizoma panacis majoris, Rhizoma Panacis Japonici, Radix Amebiae, Herba Violae, Folium Perillae and Trachycarpus fortunei, extracts thereof, as well as Armoracia rusticana powder, locust bean gum, guar gum, etc. are not detected.

In the above-mentioned test examples 1-3, Armoracia rusticana powder, Flos Sophorae and green tea are used as password components. However, in practical applications, a manufacturer of the subject matter can choose one or more plant ingredients and plant extract components as the password components according to their own needs, and then replace them according to a certain cycle, so it is difficult for the third party to replicate successfully within a certain time limit.

Because the patches of macula are caused by the overgrowth of the aerial hyphae at the macula position, the culture material in the present application also needs to ensure the uniform growth of the aerial hyphae of basidiomycetes.

In summary, according to the mycelium packaging material having the anti-counterfeiting function and the method for using the same, the mycelium packaging material having the anti-counterfeiting function has good compression and cushioning performances, and plays a role in shock absorption and cushioning for the packaged subject matter; after the subject matter is unpacked, the mycelium packaging material cannot be restored; ordinary consumers can judge whether the subject matter is certified by means of indexes, such as whether the appearance of the material is smooth and flat, whether the color is uniform, whether the surface mycelium is white and dense, whether the structure is intact without damage, and whether it is not easy to be broken; and a manufacturer of the subject matter can choose to add different password components according to different batches, so that the mycelium packaging material is difficult to be copied, and at the same time, whether the subject matter is certified is judged by detecting whether the mycelium packaging material contains the password components correspondingly added in this batch. The mycelium packaging material is eco-friendly, and free of any harmful substances to the human body and the environment, can be decomposed by microorganisms in the soil about 90 days after landfilling, and can increase the soil fertility after degradation. The mycelium packaging material having the anti-counterfeiting function and the method for using the same are simple, low in energy consumption and low in cost. The technical solutions of the present application not only solve two problems that the existing anti-counterfeiting technology is easy to be copied and imitated in batches and cumbersome in detection means, but also solve the problem that about 170 million tons of crop straws in China are not effectively treated and incinerated every year to cause serious air pollution.

The above are only the preferred examples of the present application, and are not intended to limit the patent scope of the present application. Any equivalent structure or equivalent process transformation made by using the contents of the description and drawings of the present application, or directly or indirectly applied in other related technical fields, are similarly included in the scope of patent protection of the present application.

## Claims

1. A mycelium packaging material having an anti-counterfeiting function, which is formed by growing and twisting a culture material, wherein the culture material is formed by mixing a password component, a basidiomycete strain and a biological raw material;
the biological raw material comprises: 25-80 parts by weight of a wood fiber, 1-10 parts by weight of an auxiliary component, 5-35 parts by weight of a nutritional component and water;
the auxiliary component consists of 1-5 parts by weight of quicklime and 1-5 parts by weight of gypsum; the nutritional component consists of 5-25 parts by weight of wheat bran and 0-15 parts by weight of com flour;
the addition amount of water is 1.2-1.4 times the total weight of the wood fiber, the auxiliary component and the nutritional component; and the password component can be detected in the mycelium packaging material having the anti-counterfeiting function, and the addition amount of the password component is 0.1-10% of the total weight of the biological raw material.

2. The mycelium packaging material having the anti-counterfeiting function according to claim 1, wherein the password component is one or more of a plant or a plant extract; the plant is a food-induced and/or drug-induced plant, and has a particle size of 0-1 cm; the plant extract is a food-induced and/or drug-induced plant extract; and the addition amount of the password component is 0.5-5% of the total weight of the biological raw material.

3. The mycelium packaging material having the anti-counterfeiting function according to claim 2, wherein the plant is one or more of green tea, black tea, yellow tea, white tea, dark green tea, black tea, Fagopyrum tataricum, Folium Nelumbinis, Phyllostachys nigra, Taxus chinensis, Armoracia rusticana, Camptotheca acuminata, Catharanthus roseus, Rauvolfia verticillata, Styrax benzoin, Fructus Anisi Stellatim, Rhizoma Cynanchi Stauntonii, Radix Paeoniae Alba, Atractylodes macrocephala, Pulsatilla chinensis, Cortex Dictamni, Radix Angelicae Dahuricae, Radix Isatidis, Fructus Piperis Longi, Herba Polygoni Avicularis, Areca catech, Herba Menthae, Psoraleae Fructus, Atractylodes Lancea, Alpinia katsumadai Hayata , Folium Aconiti Kusnezoffii, Radix Bupleuri, Semen Plantaginis, Pericarpium Citri Reticulatae, Radix Paeoniae Rubra, Caulis Clematidis Armandii, Radix Vladimiriae, Herba Andrographitis, Ailanthi Cortex, Acanthopanax senticosus, Paris polyphylla, Radix et Rhizoma Rhei, Herba Cirsii Japonici Radix Cirsii Japonici, Folium Isatidis, Caulis Sargentodoxae, Cortex Illicii, Fructus Kochiae Scopariae, Radix Rehmanniae, Radix Sanguisorbae, Herba Belladonnae, Caulis Erycibes, Syringa vulgaris, Malvae Fructus, Alpinia katsumadai Hayata, Angelicae Pubescentis Radix, Cortex Eucommiae, Clinopodii Herba, Radix Changii, Folium Sennae, Radix Saposhnikoviae, Radix Stephaniae Tetrandrae, Rhizoma Dioscoreae Septemlobae, Fructus Citri Sarcodactylis, Poria cocos, Rhizoma Zingiberis, Radix Glycyrrhizae, Rhizoma Ligustici, Radix Puerariae Lobatae, Ramulus Uncariae Cum Uncis, Rhizoma Drynariae, Pericarpium Trichosanthis, Desmodium styracifolium, Cinnamomi Ramulus, seaweed, Fructus Chebulae, Albizia julibrissin, Fallopia multiflora, Ginseng Radix Rubra, Rhodiola rosea, Radix knoxiae, Fructus Galangae, Flos Carthami, Hedysari Radix, Cortex Magnoliae Officinalis, Rhizoma Picrorhizae, Pipemigrum, Bulbus Fritillariae Hupehensis, Semen Trigonellae, Viscum coloratum, Rhizoma Polygoni Cuspidati, Zanthoxylum bungeanum Maxim, Citri Grandis Exocarpium, Flos Sophorae, Phellodendri Chinensis Cortex, Rhizoma Coptidis, Radix Astragali, Radix Scutellariae, Fructus Cannabis, Celosia cristata, Herba Abri, Rhizoma Curcumae Longae, Lignum Dalbergiae Odoriferae, Gardeniae Fructus Praeparatus, Sinapis Semen, Radix Tinosporae, Fagopyri Dibotryis Rhizoma, Lonicera JapanicaThunb, Rosae Laevigatae Fructus, Calyx seu Fructus Physalis, Schizonepetae Herba, Murraya exotica, Platycodonis Radix, Flos Chrysanthemi, Exocarpium Citri Rubrum, Herba Selaginellae, Semen Cassiae, Radix Sophorae Fiavescentis, Thallus Laminariae, Semen Raphani, Glechoma longituba, Forsythia suspensa, Receptaculum Nelumbinis, Semen Nelumbinis, Zanthoxylum nitidum, Folium polygoni Tinctorll, Campsis grandiflora, Rhapontici Radix, Rhizoma Phragmitis, Fructus Momordicae, Herba Ephedrae, Verbena officinali, Portulacae Herba, Aristolochia debilis, Rhododendri Daurici Folium, Fructus Viticis, Terminalia chebula, Cortex Moutan, Herba Echiptae, Chaenomelis Fructus, Semen Oroxyli, Radix Aucklandiae, Radix et Rhizoma Baphicacanthi, Radix Adenophorae, Fructus Schisandrae Sphenantherae, Fructus Arctii, Achyranthes bidentata Blume, Semen Sterculiae Lychnophorae, Eupatorii Herba, Taraxacum mongolicum, Pollen Typhae Angustifoliae, Herba Dianthi, Homalomena occulta, Radix Peucedani, Radix Rubiae, Radix Gentianae Macrophyllae, Cortex Fraxini, Indigo Naturalis, Caulis Sinomenii, Artemisia carvifolia, Radix Aucklandiae, Pericarpium Citri Reticulatae Viride, Herba Swertiae Mileensis, Rhizoma Bistortae, Ginseng Radix et Rhizoma, Folium Ginseng, Herba cistanches, Cinnamomum cassia Presl, Saururus chinensis, Notoginseng Radix Et Rhizoma, cortex mori, Herba Taxilli, Mori Folium, Hippophae, Semen Astragali Complanati, Rhizoma Kaempferiae, Rhizoma Belamcandae, Herba Lycopodii, Cimicifugae Rhizoma, Rhizoma Acori Tatarinowii, Pericarpium Granati, Calyx Kaki, Polygoni Multiflori Caulis, Lignum Sappan, Semen Ziziphi spinosae, Herba Cynomorii, Radix Pseudstellariae, Trichosanthis Radix, Radix Semiaquilegiae, Bulbus Fritillariae Thunbergii, Radix Inulae Helenii, Santali Albi Lignum, Potentillae Chinensis Herba, Radix Linderae, Croci Stigma, Radix Panacis Quinquefolii, Spica Prunellae, Corydalis Decumbentis Rhizoma, Cyperi Rhizoma, Foeniculi Fructus, Herba Cirsii Setosi, Bulbus Allii Macrostemonis, Flos Magnoliae, Radix Cynanchi Paniculati, Flos Inulae, Herba Cissampelotis, Linum usitatissimum, Rhizoma Corydalis, Flos Chrysanthemi Indici, Fritillariae Pallidiflorae Bulbus, Leonuri Herba, Herba Epimedii, Radix Stellariae, Folium Ginkgo, Houttuynia cordata Thunb, Pruni Semen, Polygalae Radix, Rhizoma Acori Calami, Bulbus Fritillariae Thunbergii, Rhizoma Anemarrhenae, Gardeniae Fructus, Aurantii Fructus, Fructus Aurantii Immaturus, Sarcandrae Herba, Rhizoma panacis majoris, Rhizoma Panacis Japonici, Radix Arnebiae, Herba Violae, Folium Perillae and Trachycarpus fortunei; and the plant extract is one or more of Armoracia rusticana powder, locust bean gum, guar gum, a green tea extract, a black tea extract, a yellow tea extract, a white tea extract, a dark green tea extract, a black tea extract, a Fagopyrum tataricum extract, a Folium Nelumbinis extract, a Phyllostachys nigra extract, a Taxus chinensis extract, a Camptotheca acuminata extract, a Catharanthus roseus extract, a Rauvolfia verticillate extract, a Styrax benzoin extract, a Fructus Anisi Stellatim extract, a Rhizoma Cynanchi Stauntonii extract, a Radix Paeoniae Alba extract, an Atractylodes macrocephala extract, a Pulsatilla chinensis extract, a Cortex Dictamni extract, a Radix Angelicae Dahuricae extract, a Radix Isatidis extract, a Fructus Piperis Longi extract, a Herba Polygoni Avicularis extract, an Areca catechu extract, a Herba Menthaeextract, a Psoraleae Fructus extract, an Atractylodes Lancea extract, a Alpinia katsumadai Hayata extract, a Folium Aconiti Kusnezoffii extract, a Radix Bupleuri extract, a Semen Plantaginis extract, a Pericarpium Citri Reticulatae extract, a Radix Paeoniae Rubra extract, a Caulis Clematidis Armandii extract, a Radix Vladimiriae extract, a Herba Andrographitis extract, an Ailanthi Cortex extract, an Acanthopanax senticosus extract, a Paris polyphylla extract, a Radix et Rhizoma Rhei extract, a Herba Cirsii Japonici Radix Cirsii Japonici extract, a Folium Isatidis extract, a Caulis Sargentodoxae extract, a Cortex Illicii extract, a Fructus Kochiae Scopariae extract, a Radix Rehmanniae extract, a Radix Sanguisorbae extract, a Herba Belladonnae extract, a Caulis Erycibes extract, a Syringa vulgaris extract, a Malvae Fructus extract, an Alpinia katsumadai Hayata extract, an Angelicae Pubescentis Radix extract, a Cortex Eucommiae extract, a Clinopodii Herba extract, a Radix Changii extract, a Folium Sennae extract, a Radix Saposhnikoviae extract, a Radix Stephaniae Tetrandrae extract, a Rhizoma Dioscoreae Septemlobae extract, a Fructus Citri Sarcodactylis extract, a Poria cocos extract, a Rhizoma Zingiberis extract, a Radix Glycyrrhizae extract, a Rhizoma Ligustici extract, a Radix Puerariae Lobatae extract, a Ramulus Uncariae Cum Uncis extract, a Rhizoma Drynariae extract, a Pericarpium Trichosanthis extract, a Desmodium styracifolium extract, a Cinnamomi Ramulus extract, a seaweed extract, a Fructus Chebulae extract, an Albizia julibrissin extract, a Fallopia multiflora extract, a Ginseng Radix Rubra extract, a Rhodiola rosea extract, a Radix knoxiae extract, a Fructus Galangae extract, a Flos Carthami extract, a Hedysari Radix extract, a Cortex Magnoliae Officinalis extract, a Rhizoma Picrorhizae extract, a Pipemigrum extract, a Bulbus Fritillariae Hupehensis extract, a Semen Trigonellae extract, a Viscum coloratum extract, a Rhizoma Polygoni Cuspidati extract, a Zanthoxylum bungeanum Maxim extract, a Citri Grandis Exocarpium extract, a Flos Sophorae extract, a Phellodendri Chinensis Cortex extract, a Rhizoma Coptidis extract, a Radix Astragali extract, a Radix Scutellariae extract, a Fructus Cannabis extract, a Celosia cristata extract, a Herba Abri extract, a Rhizoma Curcumae Longae extract, a Lignum Dalbergiae Odoriferae extract, a Gardeniae Fructus Praeparatus extract, a Sinapis Semen extract, a Radix Tinosporae extract, a Fagopyri Dibotryis Rhizoma extract, a Lonicera JapanicaThunb extract, a Rosae Laevigatae Fructus extract, a Calyx seu Fructus Physalis extract, a Schizonepetae Herba extract, a Murraya exotica extract, a Platycodonis Radix extract, a Flos Chrysanthemi, Exocarpium Citri Rubrum extract, a Herba Selaginellae extract, a Semen Cassiae extract, a Radix Sophorae Fiavescentis extract, a Thallus Laminariae extract, a Semen Raphani extract, a Glechoma longituba extract, a Forsythia suspensa extract, a Receptaculum Nelumbinis extract, a Semen Nelumbinis extract, a Zanthoxylum nitidum extract, a Folium polygoni Tinctorll extract, a Campsis grandiflora extract, a Rhapontici Radix extract, a Rhizoma Phragmitis extract, a Fructus Momordicae extract, a Herba Ephedrae extract, a Verbena officinali extract, a Portulacae Herba extract, an Aristolochia debilis extract, a Rhododendri Daurici Folium extract, a Fructus Viticis extract, a Terminalia chebula extract, a Cortex Moutan extract, a Herba Echiptae extract, a Chaenomelis Fructus extract, a Semen Oroxyli extract, a Radix Aucklandiae extract, a Radix et Rhizoma Baphicacanthi extract, a Radix Adenophorae extract, a Fructus Schisandrae Sphenantherae extract, a Fructus Arctii extract, an Achyranthes bidentata Blume extract, a Semen Sterculiae Lychnophorae extract, a Eupatorii Herba extract, a Taraxacum mongolicum extract, a Pollen Typhae Angustifoliae extract, a Herba Dianthi extract, a Homalomena occulta extract, a Radix Peucedani extract, a Radix Rubiae extract, a Radix Gentianae Macrophyllae extract, a Cortex Fraxini extract, an Indigo Naturalis extract, a Caulis Sinomenii extract, an Artemisia carvifolia extract, a Radix Aucklandiae extract, a Pericarpium Citri Reticulatae Viride extract, a Herba Swertiae Mileensis extract, a Rhizoma Bistortae extract, a Ginseng Radix et Rhizoma extract, a Folium Ginseng extract, a Herba cistanches extract, a Cinnamomum cassia Presl extract, a Saururus chinensis extract, a Notoginseng Radix Et Rhizoma extract, a cortex mori extract, a Herba Taxilli extract, a Mori Folium extract, a Hippophae extract, a Semen Astragali Complanati extract, a Rhizoma Kaempferiae extract, a Rhizoma Belamcandae extract, a Herba Lycopodii extract, a Cimicifugae Rhizoma extract, a Rhizoma Acori Tatarinowii extract, a Pericarpium Granati extract, a Calyx Kaki extract, a Polygoni Multiflori Caulis extract, a Lignum Sappan extract, a Semen Ziziphi spinosae extract, a Herba Cynomorii extract, a Radix Pseudstellariae extract, a Trichosanthis Radix extract, a Radix Semiaquilegiae extract, a Bulbus Fritillariae Thunbergii extract, a Radix Inulae Helenii extract, a Santali Albi Lignum extract, a Potentillae Chinensis Herba extract, a Radix Linderae extract, a Croci Stigma extract, a Radix Panacis Quinquefolii extract, a Spica Prunellae extract, a Corydalis Decumbentis Rhizoma extract, a Cyperi Rhizoma extract, a Foeniculi Fructus extract, a Herba Cirsii Setosi extract, a Bulbus Allii Macrostemonis extract, a Flos Magnoliae extract, a Radix Cynanchi Paniculati extract, a Flos Inulae extract, a Herba Cissampelotis extract, a Linum usitatissimum, Rhizoma Corydalis extract, a Flos Chrysanthemi Indici extract, a Fritillariae Pallidiflorae Bulbus extract, a Leonuri Herba extract, a Herba Epimedii extract, a Radix Stellariae extract, a Folium Ginkgo extract, a Houttuynia cordata Thunb extract, a Pruni Semen extract, a Polygalae Radix extract, a Rhizoma Acori Calami extract, a Bulbus Fritillariae Thunbergii extract, a Rhizoma Anemarrhenae extract, a Gardeniae Fructus extract, an Aurantii Fructus extract, a Fructus Aurantii Immaturus extract, a Sarcandrae Herba extract, a Rhizoma panacis majoris extract, a Rhizoma Panacis Japonici extract, a Radix Amebiae extract, a Herba Violae extract, a Folium Perillae extract and a Trachycarpus fortune extract.

4. The mycelium packaging material having the anti-counterfeiting function according to any one of claims 1 to 3, wherein the basidiomycete strain is one of oyster mushroom, pleurotus nebrodensis, Ganoderma lucidum, shiitake mushroom, straw mushroom, enoki mushroom and trametes gibbosa, and has an inoculation amount of 10-30% of the total weight of the biological raw material; the wood fiber is one or more of hemp stalks, wheat straws, corn straws, miscellaneous sawdust, and soybean straws; the wood fiber has a particle size of 0-1 cm (excluding an endpoint value 0); and the wood fiber consists of 15-50 parts by weight of hemp stalks, 0-15 parts by weight of wheat straws, 15-40 parts by weight of miscellaneous sawdust, 0-35 parts by weight of corn straws, and 0-20 parts by weight of soybean straws.

5. A method for using the mycelium packaging material having the anti-counterfeiting function according to any one of claims 1 to 4, comprising the following steps which are implemented in sequence:
S1: preparation of culture material: first preparing a biological raw material according to a formula, mixing well and then sterilizing; inoculating a basidiomycete strain; and finally mixing a password component into the inoculated biological raw material, and mixing well to obtain the culture material; and
S2: molding culture: putting the culture material and a subject matter together in a breathable and waterproof sterile mold such that the culture material evenly wraps an outer wall of the subject matter, and finally culturing in the dark for 5-8 days under the conditions that the temperature is 20-30°C and the humidity is 90-100% to obtain an anti-counterfeiting subject matter.

6. The method for using the mycelium packaging material having the anti-counterfeiting function according to claim 5, further comprising a step S3:
S3: anti-counterfeiting detection: first observing whether a material around a suspected subject matter is smooth and flat, whether the color is uniform, whether a surface mycelium is white and dense, and whether the structure is intact; then detecting physical properties of the material around the suspected subject matter; and finally taking the material around the suspected subject matter, selecting a corresponding detection method according to the password component added in the corresponding batch, and detecting whether the material contains the added password component.

7. The method for using the mycelium packaging material having the anti-counterfeiting function according to claim 5 or 6, wherein before the culture material wraps the outer wall of the subject matter, a filler block is placed on the outer wall of the subject matter corresponding to a trademark position; and after the culture is completed, the filler block is taken away.

8. The method for using the mycelium packaging material having the anti-counterfeiting function according to claim 7, wherein after the culture is completed, the culture material is also air-dried in natural air until the water content of the mycelium packaging material having the anti-counterfeiting function is less than 15%.

9. The method for using the mycelium packaging material having the anti-counterfeiting function according to claim 8, wherein during the preparation of the biological raw materials, wood fiber and an auxiliary component are first mixed with water and then stacked and fermented for 18-24 h, and then added with a nutritional component.

10. The method for using the mycelium packaging material having the anti-counterfeiting function according to claim 9, wherein the biological raw material is autoclaved for 1-2 h under the conditions that the temperature is 121-126°C and the pressure is 0.12-0.15 MPa; and the password component is also sterilized before addition.
